# EUROPEAN PATENT APPLICATION

(11) **EP 4 015 615 A1**
(43) Date of publication of application: **22.06.2022**
(21) Application number: 20867351.7
(22) Date of filing: 11.05.2020
(51) Int. Cl.: C12M 1/00, G16B 40/00

(54) **INFORMATION PROCESSING DEVICE, INFORMATION PROCESSING METHOD, AND INFORMATION PROCESSING PROGRAM**

(30) Priority: 24.09.2019 JP 2019173365
(71) Applicant: FUJIFILM Corporation, Tokyo 106-8620 (JP)
(72) Inventor: NAKAMURA, Naoki, Ashigarakami-gun, Kanagawa 258-8577 (JP); HARAGUCHI, Nobuyuki, Ashigarakami-gun, Kanagawa 258-8577 (JP)
(74) Representative: Hoffmann Eitle
(86) International application number: PCT/JP2020/018809
(87) International publication number: WO 2021/059578

(57) **Abstract**

An information processing apparatus estimates a quality of an antibody produced from cells and a quality of the cells on the basis of a culture state of the cells, searches for the culture state of the cells that improves the estimated quality of the antibody and the estimated quality of the cells, and derives process conditions for cell culture in which a culture state of the cells is the searched culture state.

## Description

### BACKGROUND OF THE INVENTION

### 1. Field of the Invention

The present disclosure relates to an information processing apparatus, an information processing method, and an information processing program.

### 2. Description of the Related Art

JP2009-44974A discloses a method for constructing an estimation model for estimating a quality of cells. In the method, for two or more samples in which cells of the same species are cultured, images are acquired by capturing images of cells of each sample are captured at two or more time points with different culture times, and each acquired image is analyzed, thereby generating numerical data for two or more indicators of morphology of the cells. In the method, actual measurement data of the estimation target is provided for each sample, the generated numerical data is used as an input value, and the provided actual measurement data is used as a teacher value for fuzzy neural network analysis, thereby building an estimation model that indicates a combination of indicators effective for estimation that calculate an output value on the basis of the fuzzy rule.

### SUMMARY OF THE INVENTION

Perfusion culture is a culture method of cells used in the production of biopharmaceutical drugs using antibodies produced from cells. Perfusion culture is a culture method in which a culture liquid containing cells is continuously filtered and discharged, while a fresh culture medium containing nutritional components is continuously supplied to a culture tank. Perfusion culture is also referred to as continuous culture.

Since the number of process conditions for cell cultures that can be changed in perfusion culture and the set values of each process condition are very large, it is difficult to experiment with all combinations to find the optimum process conditions. Therefore, cell culture is performed under a certain number of different process conditions, and the process conditions with the most desirable experimental results are selected. The selected process condition is optimal among the process conditions within the range of experiments, but more suitable process conditions may exist. In a case where such appropriate process conditions can be derived, it is possible to effectively support perfusion culture.

The technique described in JP2009-44974A estimates the quality of cells from images obtained by capturing cells at two different time points using an estimation model, and does not derive process conditions.

The present disclosure has been made in view of the above-mentioned circumstances, and provides an information processing apparatus, an information processing method, and an information processing program capable of effectively supporting perfusion culture.

The information processing apparatus of the present disclosure comprises: an estimation unit that estimates a quality of an antibody produced from cells and a quality of the cells on the basis of a culture state of the cells, a search unit that searches for the culture state of the cells, which improves the quality of the antibody and the quality of the cells estimated by the estimation unit, and a derivation unit that derives process conditions for cell culture in which a culture state of the cells is the culture state searched by the search unit.

In the information processing apparatus of the present disclosure, the culture state may include the number of the cells, a pH of a culture medium, a concentration of a dissolved gas in the culture medium, and a gas transfer capacity coefficient of the culture medium.

Further, in the information processing apparatus of the present disclosure, the process conditions may include a rotation speed of a stirring device, which is for stirring the culture medium, per unit time and a gas aeration amount of the culture medium per unit volume.

Further, in the information processing apparatus of the present disclosure, the estimation unit may estimate the quality of the antibody and the quality of the cells, on the basis of the culture state of the cells and a trained model which is trained in advance using the culture state, the quality of the antibody, and the quality of the cells.

Further, in the information processing apparatus of the present disclosure, the derivation unit may derive the process conditions, on the basis of the culture state of the cells and a trained model which is trained in advance using the process conditions and the culture state.

Further, in the information processing apparatus of the present disclosure, the search unit may search for the culture state of the cells in accordance with a predetermined search algorithm.

Further, in the information processing apparatus of the present disclosure, the search algorithm may be a genetic algorithm.

Further, the information processing method of the present disclosure executed by a computer, the method comprises: estimating a quality of an antibody produced from cells and a quality of the cells on the basis of a culture state of the cells; searching for the culture state of the cells that improves the estimated quality of the antibody and the estimated quality of the cells; and deriving process conditions for cell culture in which a culture state of the cells is the searched culture state.

In addition, the information processing program of the present disclosure causes a computer to execute processing of: estimating a quality of an antibody produced from cells and a quality of the cells on the basis of a culture state of the cells; searching for the culture state of the cells that improves the estimated quality of the antibody and the estimated quality of the cells; and deriving process conditions for cell culture in which a culture state of the cells is the searched culture state.

According to the present disclosure, it is possible to effectively support perfusion culture.

### BRIEF DESCRIPTION OF THE DRAWINGS

Fig. 1 is a diagram showing an example of a configuration of a cell culture device.
Fig. 2 is a diagram for explaining a flow of development of an antibody drug.
Fig. 3 is a block diagram showing an example of a hardware configuration of an information processing apparatus.
Fig. 4 is a diagram showing an example of first learning data.
Fig. 5 is a diagram for explaining the first learning data.
Fig. 6 is a diagram showing an example of second learning data.
Fig. 7 is a diagram for explaining the second learning data.
Fig. 8 is a block diagram showing an example of a functional configuration in a learning phase of the information processing apparatus.
Fig. 9 is a diagram showing an example of a first trained model.
Fig. 10 is a diagram showing an example of a second trained model.
Fig. 11 is a flowchart showing an example of learning processing.
Fig. 12 is a block diagram showing an example of a functional configuration in an operation phase of the information processing apparatus.
Fig. 13 is a diagram showing an example of a processing flow in an operation phase of the information processing apparatus.
Fig. 14 is a diagram for explaining crossover of individuals in a genetic algorithm.
Fig. 15 is a diagram for explaining mutations in the genetic algorithm.
Fig. 16 is a diagram showing an example of a process condition display screen.
Fig. 17 is a flowchart showing an example of process condition derivation processing.

### DESCRIPTION OF THE PREFERRED EMBODIMENTS

Examples of embodiments for carrying out the technique of the present disclosure will be hereinafter described in detail with reference to the drawings.

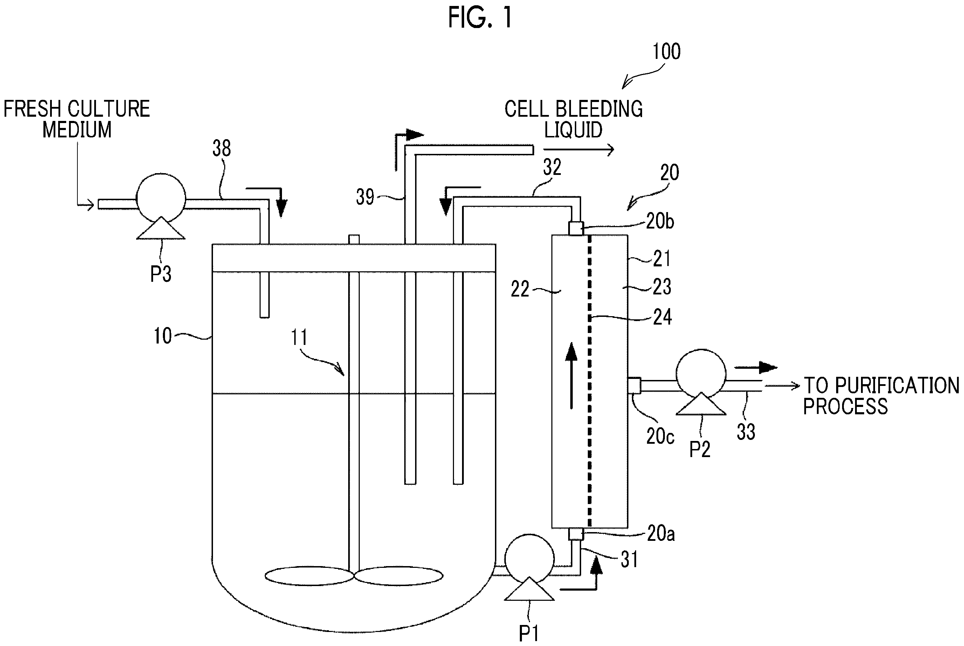

A configuration of a cell culture device 100 according to the present embodiment will be described with reference to Fig. 1. The cell culture device 100 can be suitably used in cell culture for expressing an antibody in animal cells, for example.

The cells used in expressing the antibody are not particularly limited, and examples thereof include animal cells, plant cells, eukaryotic cells such as yeast, prokaryotic cells such as grass bacillus, Escherichia coli, and the like. Animal cells such as CHO cells, BHK-21 cells, and SP2/0-Ag14 cells are preferable, and CHO cells are more preferable.

The antibody expressed in animal cells is not particularly limited, and includes, for example, anti-IL-6 receptor antibody, anti-IL-6 antibody, anti-glypican-3 antibody, anti-CD3 antibody, anti-CD20 antibody, anti-GPIIb/IIIa antibody, anti-TNF antibody, anti-CD25 antibody, anti-EGFR antibody, anti-Her2/neu antibody, anti-RSV antibody, anti-CD33 antibody, anti-CD52 antibody, anti-IgE antibody, anti-CD11a antibody, anti-VEGF antibody, anti-VLA4 antibody, and the like. The antibody includes not only monoclonal antibodies derived from animals such as humans, mice, rats, hamsters, rabbits, and monkeys, but also artificially modified antibodies such as chimeric antibodies, humanized antibodies, and bispecific antibodies.

The obtained antibody or fragment thereof can be purified to be uniform. For the separation and purification of the antibody or a fragment thereof, the separation and purification method used in a conventional polypeptide may be used. For example, an antibody can be separated and purified by appropriately selecting and combining a chromatography column such as affinity chromatography, a filter, ultrafiltration, salting out, dialysis, SDS polyacrylamide gel electrophoresis, and isoelectric point electrophoresis. However, the present invention is not limited thereto. The obtained concentration of the antibody can be measured by measurement of the absorbance or by an enzyme-linked immunosorbent assay (ELISA) or the like.

As shown in Fig. 1, the cell culture device 100 includes a culture container 10 that contains a cell suspension including cells, and a filter unit 20 that has a filter membrane 24 for subjecting the cell suspension extracted from the culture container 10 to a membrane separation treatment. The cell culture device 100 further includes a flow passage 32 as a circulation flow passage for returning components blocked by the filter membrane 24 to the culture container 10, and a flow passage 33 for discharging components having permeated through the filter membrane 24 of the cell suspension to the outside of the filter unit 20. Further, the cell culture device 100 includes a flow passage 38 for supplying a fresh culture medium to the culture container 10, and a pump P3 provided in the middle of the flow passage 38.

The culture container 10 is a container for containing a cell suspension including cells and a culture medium used in expressing an antibody. Inside the culture container 10, a stirring device 11 having a stirring blade is provided. By rotating the stirring blade of the stirring device 11, the culture medium contained together with the cells in the culture container 10 is stirred, and the homogeneity of the culture medium is maintained.

In the cell culture device 100, in order to prevent the concentration of cells in the culture container 10 from becoming excessively high, a cell bleeding treatment is performed, which is for bleeding off a part of the cells in the culture container 10 (for example, about 10%) at an appropriate timing during the culture period. In the cell bleeding treatment, the cells in the culture container 10 are discharged to the outside of the culture container 10 through the flow passage 39.

One end of the flow passage 31 is connected to the bottom of the culture container 10, and the other end is connected to an inlet 20a of the filter unit 20. In the middle of the flow passage 31, a pump P1, which extracts the cell suspension contained in the culture container 10 and sends the cell suspension to the filter unit 20, is provided.

The filter unit 20 comprises a container 21 and a filter membrane 24 that separates the space inside the container 21 into a supply side 22 and a permeation side 23 and performs a membrane separation treatment on the cell suspension extracted from the culture container 10. Further, the filter unit 20 has the inlet 20a through which the cell suspension flows in and an outlet 20b through which the cell suspension flows out on the supply side 22. The cell suspension extracted from the culture container 10 passes through the filter membrane 24 while flowing into the inside of the container 21 from the inlet 20a and flowing out to the outside of the container 21 from the outlet 20b. The filter unit 20 performs the membrane separation treatment by a tangential flow (cross flow) method of sending permeation components to the permeation side 23 while flowing a liquid subjected to the membrane separation treatment along the membrane surface of the filter membrane 24 subjected to the membrane separation treatment (that is, in a direction parallel to the membrane surface). The tangential flow method, which is a method for membrane separation treatment using the filter membrane 24, may be a method of forming a flow in which the cell suspension extracted from the culture container 10 circulates in one direction in parallel along the membrane surface of the filter membrane 24, or may be a method of forming a flow in which the cell suspension extracted from the culture container 10 reciprocates alternately in parallel along the membrane surface of the filter membrane 24. In a case of forming a circulating flow, for example, a KrosFlo perfusion culture flow path device (KML-100, KPS-200, and KPS-600) manufactured by Spectrum Laboratories Corp. can be suitably used. Further, in a case of forming a flow that reciprocates alternately, the ATF system manufactured by REPLIGEN Corp. can be suitably used.

The relatively large-sized components included in the cell suspension do not permeate through the filter membrane 24, flow out to the outside of the container 21 from the outlet 20b, and are returned to the inside of the culture container 10 through the flow passage 32. That is, in the cell suspension extracted from the culture container 10, the components blocked by the filter membrane 24 are returned to the inside of the culture container 10 through the flow passage 32. On the other hand, the relatively small-sized components included in the cell suspension permeate through the filter membrane 24 and are discharged to the outside of the container 21 from a discharge port 20c provided on the permeation side 23. A flow passage 33 provided with a pump P2 is connected to the discharge port 20c of the filter unit 20, and the components discharged to the permeation side 23 are discharged from the discharge port 20c to the outside of the container 21 through the flow passage 33.

In the cell culture device 100 according to the present embodiment, the filter membrane 24 is used for the purpose of separating cells and components unnecessary for cell culture. Examples of components unnecessary for cell culture include cell carcasses, cell crushed products, DNA, HCP, antibodies, waste products, and the like. That is, the filter membrane 24 has a separation performance suitable for blocking the permeation of cells while allowing components unnecessary for cell culture to permeate.

The components unnecessary for cell culture discharged from the culture container 10 as described above are sent to the next process, which is an antibody purification process.

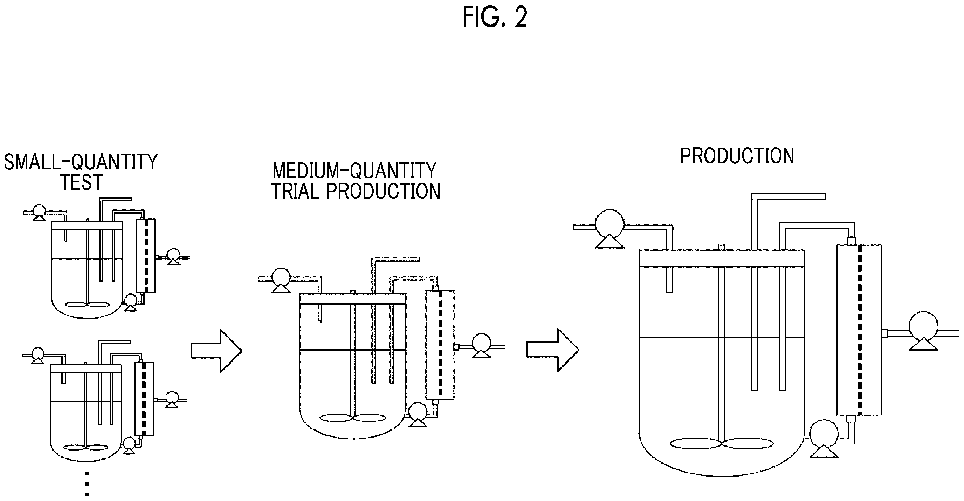

In the contract development of antibody drugs, cells are contracted from customers and antibodies are produced by culturing the contracted cells. In the contract development, as shown in Fig. 2, an appropriate process condition is determined by a small-quantity test in which perfusion culture is performed under various process conditions using a relatively small-scale cell culture device 100. Next, using a relatively medium-scale cell culture device 100, the qualities of the antibody and cells are confirmed by a medium-quantity trial production in which perfusion culture is performed under the process conditions determined by the small-quantity test. After confirmation of the qualities of the antibody and cells in the medium-quantity trial production is completed, the antibody is produced by performing perfusion culture using a relatively large-scale cell culture device 100.

In the present embodiment, an example of deriving more appropriate process conditions to be used in the next medium-quantity trial production in the above-mentioned small-quantity test will be described.

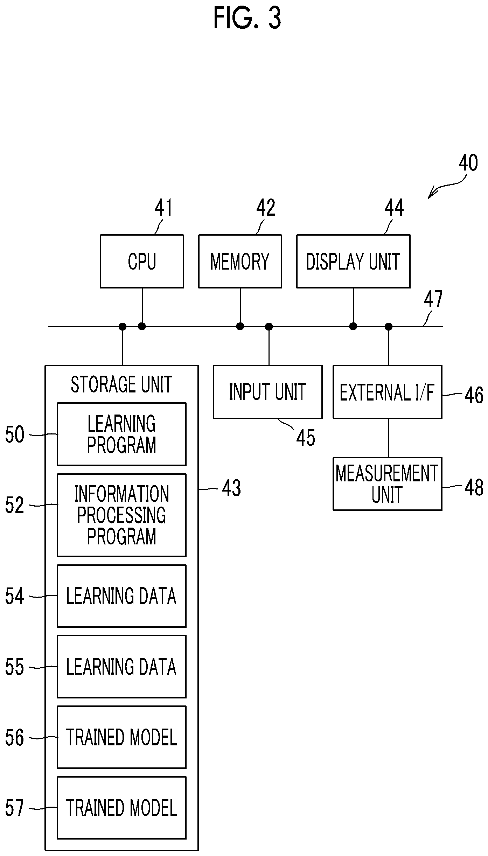

Next, referring to Fig. 3, the hardware configuration of the information processing apparatus 40 connected to the cell culture device 100 will be described. As shown in Fig. 3, the information processing apparatus 40 includes a central processing unit (CPU) 41, a memory 42, a storage unit 43, a display unit 44 such as a liquid crystal display, an input unit 45 such as a keyboard and a mouse, and an external interface (I/F) 46. The CPU 41, the memory 42, the storage unit 43, the display unit 44, the input unit 45, and the external I/F 46 are connected to the bus 47. The measurement unit 48 is connected to the external I/F 46. Examples of the information processing apparatus 40 include a personal computer, a server computer, and the like.

The storage unit 43 is realized by a hard disk drive (HDD), a solid state drive (SSD), a flash memory, or the like. A learning program 50 and an information processing program 52 are stored in the storage unit 43 as a storage medium. The CPU 41 reads the learning program 50 from the storage unit 43, expands the program into the memory 42, and executes the expanded learning program 50. Further, the CPU 41 reads the information processing program 52 from the storage unit 43, expands the program into the memory 42, and executes the expanded information processing program 52. Further, the storage unit 43 stores first learning data 54 and second learning data 55. Further, the storage unit 43 stores the first trained model 56 and the second trained model 57.

The measurement unit 48 includes various measurement devices that measure a culture state of cells in the cell culture using the cell culture device 100. Examples of the culture state include: for example, the number of cells contained in the culture container 10 (hereinafter, simply referred to as "number of cells"); a pH of the culture medium; a concentration of the dissolved gas in the culture medium (for example, a concentration of dissolved oxygen); a gas transfer capacity coefficient (for example, oxygen transfer capacity coefficient: kLA) of the culture medium; and the like. The number of cells means a sum of the number of living cells and the number of dead cells.

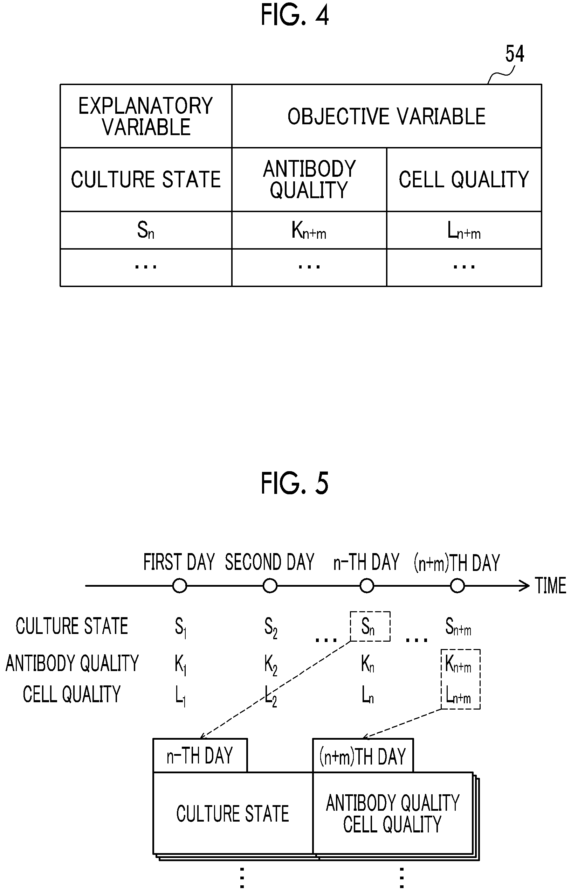

Referring to Figs. 4 and 5, the details of the learning data 54 according to the present embodiment will be described. As shown in Fig. 4, the learning data 54 is a data set for learning that includes a plurality of sets of a culture state in the cell culture which is an explanatory variable, a quality of the antibody produced from the cells which is an objective variable corresponding to the explanatory variable, and a quality of the cells. Examples of the quality of the antibody include a concentration of the antibody, an aggregate amount of the antibody, a decomposition product amount of the antibody, an immature sugar chain amount, and the like. Examples of quality of the cells include a cell survival rate and a cell viability. The quality of the antibody and the quality of the cells may be one of the index values or a combination of a plurality of index values. Further, the quality of the antibody and the quality of the cells may be evaluation values obtained by determining one or a plurality of combinations thereof in a plurality of stages (for example, four stages A to D) in accordance with a predetermined determination standard.

As shown in Fig. 5, in the present embodiment, in the past cell culture, the learning data 54 includes a culture state acquired at a time point at which a predetermined period n (hereinafter referred to as "cell proliferation period") has elapsed as a period for cell proliferation after the start of cell culture. Further, in the present embodiment, the learning data 54 also includes a quality of the antibody and a quality of the cells, which are associated with the acquired culture state, after a predetermined period m has elapsed since the culture state was acquired. For example, in a case where the small-quantity test is performed for 30 days and the cell proliferation period is 10 days, n in Figs. 4 and 5 is 10 and m is 20. It should be noted that n and m are not limited to the example. For example, n is 5 and m is 9. That is, the quality of the antibody and the quality of the cells after 14 days from the start of cell culture may be associated with the culture state after 5 days from the start of cell culture. The culture state acquired at the time point at which the cell proliferation period has elapsed is used since the culture state is often unstable during the cell proliferation period.

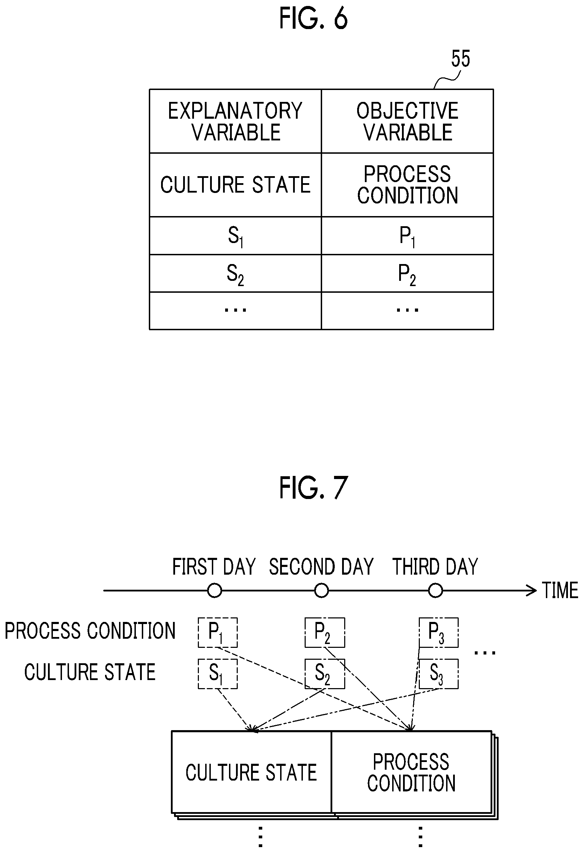

Referring to Figs. 6 and 7, the details of the learning data 55 according to the present embodiment will be described. As shown in Fig. 6, the learning data 55 is a data set for learning that includes a plurality of sets of a culture state which is an explanatory variable and a process condition in cell culture which is an objective variable corresponding to the explanatory variable. Examples of process conditions include a rotation speed of the stirring device 11 per unit time (hereinafter referred to as "stirring rotation speed"), a gas aeration amount of the culture medium contained in the culture container 10 per unit volume, and a temperature of the culture medium to be contained in the culture container 10.

As shown in Fig. 7, each data included in the learning data 55 includes process conditions acquired periodically (for example, once a day) and culture states of the cells which is cultured under the process conditions, in the past cell culture.

The trained model 56 is a model that is trained in advance using the learning data 54, and the trained model 57 is a model that is trained in advance using the learning data 55. Examples of the trained model 56 and the trained model 57 include a neural network model. The trained model 56 and the trained model 57 are generated by the information processing apparatus 40 in the learning phase to be described later.

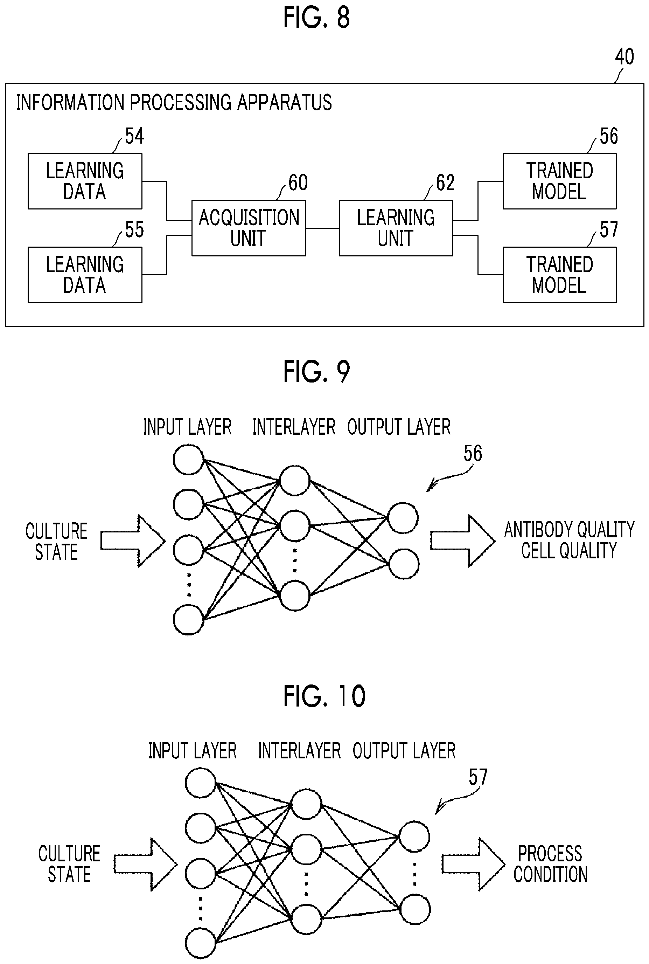

Next, referring to Fig. 8, a functional configuration in the learning phase of the information processing apparatus 40 will be described. As shown in Fig. 8, the information processing apparatus 40 includes an acquisition unit 60 and a learning unit 62. In a case where the CPU 41 executes the learning program 50, the CPU 41 functions as the acquisition unit 60 and the learning unit 62.

The acquisition unit 60 acquires the learning data 54 and the learning data 55 from the storage unit 43. The learning unit 62 generates the trained model 56 by training the model using the learning data 54 acquired by the acquisition unit 60 as training data. Then, the learning unit 62 stores the generated trained model 56 in the storage unit 43.

As an example, as shown in Fig. 9, the learning performed by the learning unit 62 generates a trained model 56 in which the culture state is input and the quality of the antibody and the quality of the cells are output. For example, an error back propagation method is used in learning performed by the learning unit 62. The trained model 56 may be a deep neural network model having a plurality of interlayers. Further, as the trained model 56, a model other than the neural network may be applied.

Further, the learning unit 62 generates the trained model 57 by training the model using the learning data 55 acquired by the acquisition unit 60 as training data. Then, the learning unit 62 stores the generated trained model 57 in the storage unit 43.

As an example, as shown in Fig. 10, learning performed by the learning unit 62 generates a trained model 57 in which the culture state is an input and the process conditions are an output. For example, an error back propagation method is used in learning performed by the learning unit 62. The trained model 57 may be a deep neural network model having a plurality of interlayers. Further, as the trained model 57, a model other than the neural network may be applied.

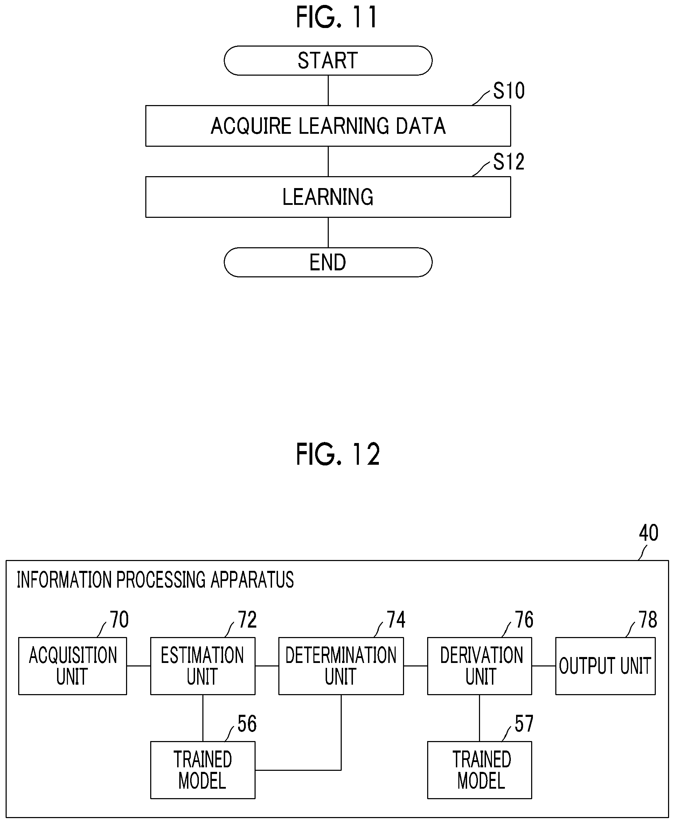

Next, referring to Fig. 11, the operation of the information processing apparatus 40 according to the present embodiment in the learning phase will be described. In a case where the CPU 41 executes the learning program 50, the learning processing shown in Fig. 11 is executed.

In step S10 of Fig. 11, the acquisition unit 60 acquires the learning data 54 and the learning data 55 from the storage unit 43. In step S12, as described above, the learning unit 62 generates the trained model 56 by training the model using the learning data 54 acquired in step S10 as the training data. Then, the learning unit 62 stores the generated trained model 56 in the storage unit 43.

Further, as described above, the learning unit 62 generates the trained model 57 by training the model using the learning data 55 acquired in step S10 as the training data. Then, the learning unit 62 stores the generated trained model 57 in the storage unit 43. In a case where step S12 ends, the learning processing ends.

Next, referring to Fig. 12, a functional configuration in the operation phase of the information processing apparatus 40 according to the present embodiment will be described. As shown in Fig. 12, the information processing apparatus 40 includes an acquisition unit 70, an estimation unit 72, a search unit 74, a derivation unit 76, and an output unit 78. In a case where the CPU 41 executes the information processing program 52, the CPU 41 functions as an acquisition unit 70, an estimation unit 72, a search unit 74, a derivation unit 76, and an output unit 78.

The acquisition unit 70 acquires the culture state of the cells in the cell culture using the cell culture device 100, which was measured by the measurement unit 48 at the time point at which the cell proliferation period has elapsed. In the present embodiment, the acquisition unit 70 acquires the culture state from each of the plurality of cell culture devices 100 in the small-quantity test.

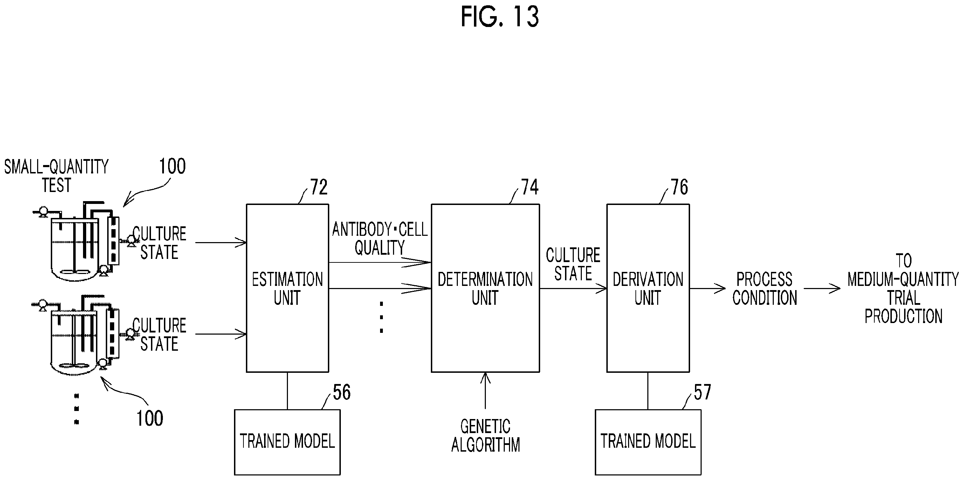

The estimation unit 72 estimates a quality of the antibody produced from the cells and a quality of the cells, on the basis of the trained model 56 and the culture state acquired by the acquisition unit 70. Specifically, the estimation unit 72 inputs the culture state acquired by the acquisition unit 70 to the trained model 56. As described above, the trained model 56 is a model that is trained using the culture state as an input and the quality of the antibody and the quality of the cells after the elapse of a predetermined period m as the output. Therefore, the output from the trained model 56 is estimated values of the quality of the antibody and the quality of the cells after the predetermined period m has elapsed from the time point at which the acquisition unit 70 acquires the culture state. As described above, the estimation unit 72 estimates the final quality of the antibody and the final quality of the cells from the culture state in each cell culture device 100 in which the small-quantity test is performed (refer to also Fig. 13).

As shown in Fig. 13, the search unit 74 searches for the culture state of the cells that improves the quality of the antibody and the quality of the cells estimated by the estimation unit 72 in accordance with a predetermined search algorithm. In the present embodiment, the search unit 74 uses a genetic algorithm as the search algorithm.

Specifically, first, the search unit 74 sets the most desirable quality of the antibody and quality of the cells among the quality of the antibody and the quality of the cells estimated by the estimation unit 72 for each cell culture device 100 as evaluation standard in the genetic algorithm. Next, the search unit 74 randomly generates a group of individuals at the initial stage. The individual described herein is a culture state of the cells in cell culture.

Next, the search unit 74 derives an evaluation value of each individual. In the present embodiment, the search unit 74 inputs each individual to the trained model 56, and derives the quality of the antibody and the quality of the cells output from the trained model 56 as evaluation values of each individual.

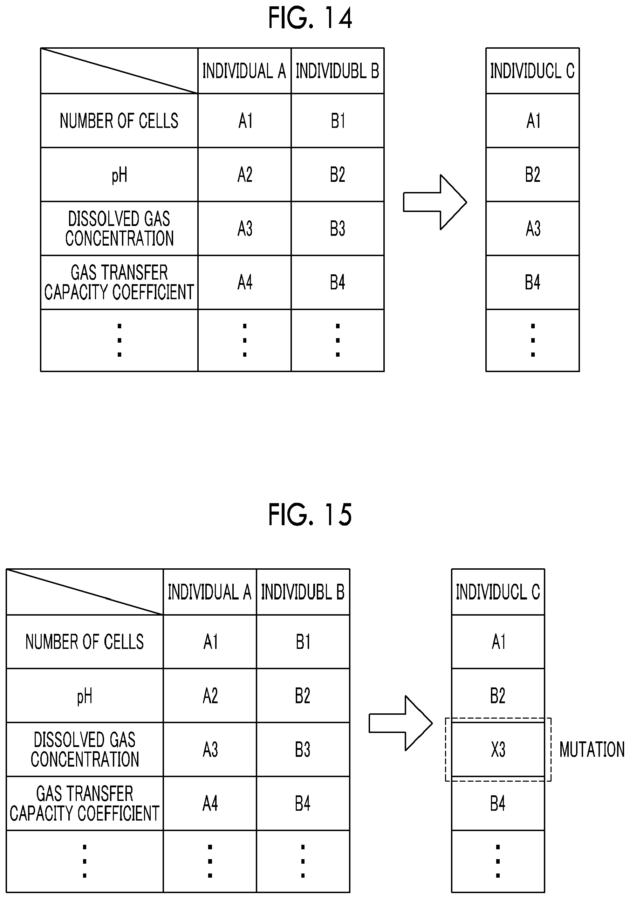

Next, as shown in Fig. 14, the search unit 74 selects two individuals and crosses the selected individuals. Further, as shown in Fig. 15, the search unit 74 generates a mutation with a certain probability for the crossed individuals. The method of selecting two individuals such as roulette selection and tournament selection, the crossing method such as two-point crossing and multi-point crossing, and the probability of occurrence of mutation are not particularly limited and may be determined experimentally in advance. The search unit 74 selects, crosses, and mutates individuals of the next generation until the number of the next generations reaches a predetermined number.

Then, the search unit 74 derives the evaluation value of each individual of the next generation by inputting each individual of the next generation to the trained model 56. The search unit 74 repeats generation of the individual of the next generation as described above until the evaluation value of the individual is greater than a set evaluation standard. Through the above-mentioned processing, the search unit 74 searches for the individual that is greater than the set evaluation standard. The individual thus searched is a culture state of the cells that improves the quality of the antibody and the quality of the cells estimated by the estimation unit 72.

As shown in Fig. 13, the derivation unit 76 derives the process conditions in which a culture state of the cells is changed to the culture state searched by the search unit 74, on the basis of the trained model 57 and the culture state searched by the search unit 74. Specifically, the derivation unit 76 inputs the culture state, which is searched by the search unit 74, to the trained model 57. From the trained model 57, the process conditions in which a culture state of the cells is the input culture state are output. The output process conditions are process conditions which are derived by the derivation unit 76.

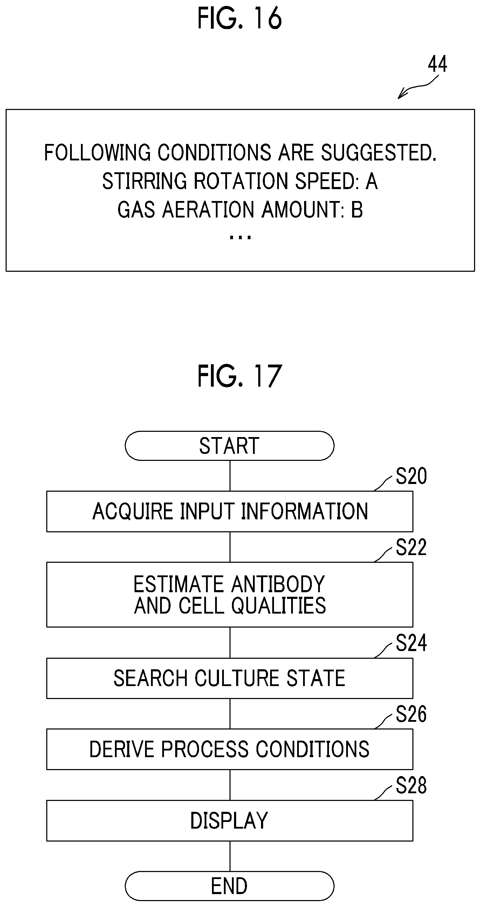

The output unit 78 displays the process conditions which are derived by the derivation unit 76 by outputting the process conditions to the display unit 44. On the basis of the output, the process condition display screen shown in Fig. 16 is displayed on the display unit 44 as an example. As shown in Fig. 16, the process conditions which are derived by the derivation unit 76 are displayed on the process condition display screen. A user confirms the displayed process conditions and uses them as process conditions in the medium-quantity trial production.

Next, referring to Fig. 17, the operation of the information processing apparatus 40 according to the present embodiment in the operation phase will be described. In a case where the CPU 41 executes the information processing program 52, the quality estimation processing shown in Fig. 17 is executed. The quality estimation processing shown in Fig. 17 is executed after the perfusion culture in the small-quantity test is started and at the timing in a case where the cell proliferation period has elapsed.

In step S20 of Fig. 17, the acquisition unit 70 acquires the culture state of the cells in the cell culture using the cell culture device 100, which is measured by the measurement unit 48 at the time point at which the cell proliferation period has elapsed. The acquisition unit 70 acquires the culture state from each of the plurality of cell culture devices 100 in the small-quantity test.

In step S22, as described above, the estimation unit 72 estimates the quality of the antibody, which is produced from the cells, and the quality of the cells, on the basis of the trained model 56 and the culture state for each of the culture states acquired in step S20. In step S24, as described above, the search unit 74 searches for the culture state of the cells that improves the quality of the antibody and the quality of the cells estimated in step S22, in accordance with a predetermined search algorithm.

In step S26, as described above, the derivation unit 76 derives the process conditions in which a culture state of the cells is the culture state searched in step S24, on the basis of the trained model 57 and the culture state which is searched in step S24. In step S28, the output unit 78 displays the process conditions which are derived in step S26 by outputting the process conditions to the display unit 44. In a case where the processing of step S28 is completed, the quality estimation processing is completed.

As described above, according to the present embodiment, the process conditions are not derived directly from the quality of the antibody and the quality of the cells, but the process conditions are derived from the quality of the antibody and the quality of the cells through the culture state. As compared with the quality of the antibody and the quality of the cells, and the process conditions, the process conditions and the culture state and the culture state and the quality of the antibody and the quality of the cells are highly related. Therefore, it is possible to derive more appropriate process conditions with high accuracy. As a result, it is possible to effectively support perfusion culture.

In the above-mentioned embodiment, the case where the genetic algorithm is applied as the search algorithm has been described, but the present invention is not limited thereto. For example, as a search algorithm, an algorithm other than a genetic algorithm such as Bayesian optimization may be applied.

Further, in the above-mentioned embodiment, the type of the cells used in learning in the learning phase and the cells used in the operation phase may be different. In such a case, for example, the trained models 56 and 57 are generated from the learning data 54 and 55 for certain cells. In such a case, in the operation phase, relatively small amounts of learning data 54 and 55 are collected for the cells used in the operation phase as compared with the learning phase. Then, the trained models 56 and 57 are subjected to retraining using the small amounts of learning data 54 and 55. Such retraining is also referred to as transfer training. By such retraining, the learning period can be shortened. At the time of the retraining, parameters such as the number of layers and the number of nodes in the interlayers of the trained models 56 and 57 may be changed.

Further, as the hardware structure of the processing unit that executes various processes such as each functional unit of the information processing apparatus 40 in the above-mentioned embodiment, it is possible to use various processors to be described below. As described above, various processors include not only a CPU as a general-purpose processor which functions as various processing units by executing software (programs) but also a programmable logic device (PLD) as a processor capable of changing a circuit configuration after manufacturing a field programmable gate array (FPGA); and a dedicated electrical circuit as a processor, which has a circuit configuration specifically designed to execute specific processing, such as an application specific integrated circuit (ASIC).

One processing unit may be configured as one of the various processors, or may be configured as a combination of two or more of the same or different kinds of processors (for example, a combination of a plurality of FPGAs or a combination of a CPU and an FPGA). Further, the plurality of processing units may be composed of one processor.

As an example of the plurality of processing units composed of one processor, first, as represented by computers such as a client and a server, there is a form in which one processor is composed of a combination of one or more CPUs and software and this processor functions as a plurality of processing units. Second, as represented by a system on chip (SoC), there is a form in which a processor that realizes the functions of the whole system including a plurality of processing units with a single integrated circuit (IC) chip is used. As described above, the various processing units are configured by using one or more of the various processors as a hardware structure.

Furthermore, as the hardware structure of these various processors, more specifically, it is possible to use an electric circuit (circuitry) in which circuit elements such as semiconductor elements are combined.

Further, in the above-mentioned embodiment, the configuration in which the learning program 50 and the information processing program 52 are stored (installed) in the storage unit 43 in advance has been described, but the present invention is not limited thereto. The learning program 50 and the information processing program 52 may be provided in a form in which the programs are stored in a storage medium such as a compact disc read only memory (CD-ROM), a digital versatile disc read only memory (DVD-ROM), and a universal serial bus (USB) memory. Further, the learning program 50 and the information processing program 52 may be downloaded from an external device through a network.

From the above-mentioned description, the technology relating to the following supplementary items can be found.

### [Additional Notes]

An information processing apparatus comprising:
a processor; and
a memory that is built into or connected to the processor,
in which the processor is configured to
estimate a quality of an antibody produced from cells and a quality of the cells on the basis of a culture state of the cells,
search for the culture state of the cells that improves the estimated quality of the antibody and the estimated quality of the cells, and
derive process conditions for cell culture in which a culture state of the cells is the searched culture state.

The present disclosure of JP2019-173365 filed on September 24, 2019 is incorporated herein by reference in its entirety. Further, all documents, patent applications, and technical standards described in the present specification are incorporated into the present specification by reference to the same extent as in a case where the individual documents, patent applications, and technical standards were specifically and individually stated to be incorporated by reference.

## Claims

1. An information processing apparatus comprising:
an estimation unit that estimates a quality of an antibody produced from cells and a quality of the cells on the basis of a culture state of the cells;
a search unit that searches for the culture state of the cells, which improves the quality of the antibody and the quality of the cells estimated by the estimation unit; and
a derivation unit that derives process conditions for cell culture in which a culture state of the cells is the culture state searched by the search unit.

2. The information processing apparatus according to claim 1, wherein the culture state includes the number of the cells, a pH of a culture medium, a concentration of a dissolved gas in the culture medium, and a gas transfer capacity coefficient of the culture medium.

3. The information processing apparatus according to claim 1 or 2, wherein the process conditions include a rotation speed of a stirring device, which is for stirring the culture medium, per unit time and a gas aeration amount of the culture medium per unit volume.

4. The information processing apparatus according to any one of claims 1 to 3, wherein the estimation unit estimates the quality of the antibody and the quality of the cells on the basis of the culture state of the cells and a trained model which is trained in advance using the culture state, the quality of the antibody, and the quality of the cells.

5. The information processing apparatus according to any one of claims 1 to 4, wherein the derivation unit derives the process conditions on the basis of the culture state of the cells and a trained model which is trained in advance using the process conditions and the culture state.

6. The information processing apparatus according to any one of claims 1 to 5, wherein the search unit searches for the culture state of the cells in accordance with a predetermined search algorithm.

7. The information processing apparatus according to claim 6, wherein the search algorithm is a genetic algorithm.

8. An information processing method executed by a computer, the method comprising:
estimating a quality of an antibody produced from cells and a quality of the cells on the basis of a culture state of the cells;
searching for the culture state of the cells that improves the estimated quality of the antibody and the estimated quality of the cells; and
deriving process conditions for cell culture in which a culture state of the cells is the searched culture state.

9. An information processing program for causing a computer to execute:
estimating a quality of an antibody produced from cells and a quality of the cells on the basis of a culture state of the cells;
searching for the culture state of the cells that improves the estimated quality of the antibody and the estimated quality of the cells; and
deriving process conditions for cell culture in which a culture state of the cells is the searched culture state.
